# EUROPEAN PATENT APPLICATION

(11) **EP 2 090 365 A1**
(43) Date of publication of application: **19.08.2009**
(21) Application number: 08150549.7
(22) Date of filing: 23.01.2008
(51) Int. Cl.: B01L 3/00, G01N 33/543

(54) **Combined cell and protein analysis on a substrate**

(71) Applicant: Koninklijke Philips Electronics N.V., 5621 BA Eindhoven (NL)
(72) Inventor: The designation of the inventor has not yet been filed
(74) Representative: Damen, Daniel Martijn

(57) **Abstract**

The present invention provides a device for capturing first and second analytes in a test sample, wherein the size of the first analyte is larger than the size of the second analyte. The device comprises (a) a chamber; (b) a porous substrate with a first surface and a second surface, said porous substrate separate the chamber into a first and a second sub-chamber; and (c) means for supplying the test sample into the first sub-chamber. The porous substrate is adapted to pass the second analyte but not to pass the first analyte and to capture the first analyte on the first surface and the second analyte on the second surface.

## Description

### FIELD OF THE INVENTION

The present invention relates a method for detecting the presence or quantity of two different analytes in a test sample by means of a flow-through assay device. In particular, the present provides a flow-through assay device with a porous membrane/substrate for detecting at least two different analytes in a fluid, wherein the particle size of the first analyte is larger than the particle size of the second analyte. The method and device of the present invention allow an all in one measurement of the two different analytes, without having to undergo sample preparation techniques. The device and method are particularly useful for detecting the presence or quantity of proteins/small-molecules and cells from a single blood sample, such as a whole blood sample.

### BACKGROUND OF THE INVENTION

In order to be detected as a biomolecule of interest, the analyte has to bind specifically to a corresponding capture probe. The analyte of interest can be brought to the capture site of the diagnostic device either by flow-over or by flow-through. These two types of sample introduction give rise to two types of assays which mainly differ in the flow direction of the analyte towards the detection sites.

In a flow-over device (often also called lateral flow assay device), the analyte flows parallel to a solid substrate. The substrate comprises an array of capture probes. The flow-over concept exhibits a low-pressure drop of the analyte flowing through the device. However, flow-over devices typically have a long assay time. In addition, a flow-over device has a relatively low sensitivity due to the limitation of the number of capture probes per projected area.

Some of these drawbacks of flow-over devices are overcome in a flow-through device in which the analyte flows perpendicularly through a porous substrate or membrane comprising immobilized capture probes. The high specific surface area of the (micro)porous substrate causes a large number of bonded probe molecules on the detection spots, resulting in a high sensitivity for the detection of the respective biomolecular analytes. More so, by pumping the analyte solution several times through the porous substrate/membrane increases the chance of the analyte: antibody binding.

In case the presence or quantity of two different analytes in a test sample should be measured, a separate flow-over or flow-through device for each of the two analytes is typically necessary. For instance, a conventional method of measuring parameters of smaller proteins and larger cells in a whole blood sample in a combined manner is always a big challenge. When looking at standard protocol followed at the central labs, it is common to categorize the white blood cells and measure different proteins (e.g. CRP, Hemoglobin) or even molecules such as cholesterol for the same request.

US-A-6,551,842 discloses a device for detecting and quantifying at least one analyte in a fluid sample suspected of containing the analyte by employing a liquid reactant capable of reacting with the analyte to form a detectable soluble reaction product. The device comprises a fluid transport material having a first zone ("fluid sample zone") for the application of the fluid sample at a fluid sample application site and a second zone ("liquid reactant zone") for the application of a liquid reactant at a liquid reactant application site, wherein when the fluid sample is added to the first zone, and the liquid reactant is added to the second zone, the fluid sample flows in a first direction from a fluid sample edge toward the second zone, and the liquid reactant flows in a second direction opposite to that of the first direction and toward the first zone from a liquid reactant edge. When the flowing fluid sample and the flowing liquid reactant meet, flow stops, the reactants diffuse toward one another, and the detectable reaction product is formed by a reaction between the liquids.

### SUMMARY OF THE INVENTION

There is a need for an easy and fast way of detecting the presence or quantity of two different types of analytes or particles, such as cells and proteins. A new device and method which provides such an easy and fast way of measuring two different types of analytes would be beneficial in diagnostic settings where the biomarkers may be of protein and cell origin.

The object is achieved with the features of the claims.

In a first aspect the invention relates to a device for capturing first (100) and second (200) analytes in a test sample, wherein the size of the first analyte (100) is larger than the size of the second analyte (200), said device comprising:
(a) a chamber (50);
(b) a porous substrate (1) with a first surface (10) and a second surface (20), said porous substrate (1) separating the chamber (50) into a first and a second sub-chamber (51 and 52, respectively); and
(c) means (41) for supplying the test sample into the first sub-chamber (51); wherein the porous substrate (1) is adapted to pass the second analyte but not to pass the first analyte and to capture the first analyte (100) on the first surface (10) and the second analyte (200) on the second surface (20).

The device and the method of the present invention are beneficial since different types of analytes or particles of different sizes, e.g. cells and proteins, can be measured simultaneously but still separated from other cell populations. The device and the method of the present invention provides the further advantage that a sample volume required from a patient is less than in conventional methods since both measurements can be done on the same sample. Furthermore, the results will be much more accurate as compared to when having two measurements done using two different techniques. In other words, the device and the method of the present invention allow a separation of components such that individual measurements can be performed.

As used herein, the term "analyte" and "particles" are used synonymously and generally refer to a substance or component to be detected in a test sample. In particular, an analyte is a substance or chemical constituent that is determined in an analytical procedure. In an immunoassay the analyte may be the ligand or the binder, while in blood glucose testing, the analyte is glucose. For instance analytes may include antigenic substances, happens, antibodies, and combinations thereof. Analytes include, but are not limited to proteins, DNA, RNA, small molecules, cells, WBC (White Blood cells, leukocytes), RBC (Red Blood Cells, erythrocytes) or bacteria, virus. Proteins, DNA, RNA and small-molecules are typically one to four (or even more) orders of magnitudes smaller than WBC, RBC and Bacteria (the diameter of a typical human erythrocyte disk is about 6-8 µm, whereas the size of an antibody molecule is about 15 nm). Analytes also include, but are not limited to, toxins, organic compounds, proteins, peptides, microorganisms, amino acids, nucleic acids, hormones, steroids, vitamins, drugs (including those administered for therapeutic purposes as well as those illicitly administered), drug intermediaries or byproducts, bacteria, virus particles and metabolites of or antibodies to any of the above substances.

As used herein, the term "test sample" generally refers to a biological material suspected of containing the analyte. The test sample may be a fluid, e.g. derived from any biological source, such as a physiological fluid, including, (whole) blood, lymph, urine, cerebral fluid, interstitial fluid, saliva, ocular lens fluid, cerebral spinal fluid, sweat, urine, milk, ascites fluid, mucous secretion, nasal fluid, sputum, synovial fluid, peritoneal fluid, vaginal fluid, menses, amniotic fluid, semen, and so forth. The sample may be collected from the patient or subjected to the analysis according to the invention. Besides physiological fluids, other liquid samples may be used, such as water, food products, and so forth, for the performance of environmental or food production assays. As used herein, the term "blood" refers to whole blood, that contains all of the blood components, or to any kind of blood product. Whole blood is usually separated into several blood products, such as red blood cells, plasma, platelets, and/or cryoprecipitate. For instance, the device and the method according to the present invention can be used to separate cell from a blood plasma.

Measuring two different analytes, e.g. cells and proteins, in one device require some sort of separation of the two parts. This is done by using a filter as a substrate. The substrate may be a porous substrate, e.g. a membrane. The first analyte, e.g. the larger cells, stay on one side of the membrane whilst the second analyte, e.g. the smaller proteins and other small molecules, are transported to the other side of the membrane. On either sides of the porous membrane, capture probes can be immobilized, e.g., antibodies which allow detection with an immunoassay and antibodies directed against the cell surfaces. By using the flow of the fluid to filter the sample, two components can be separated. Moreover, the flow of the fluid causes the components, i.e. the analytes, to be pushed in close proximity to the respective capture sides of the membrane, allowing rapid antigen antibody binding.

In the flow-through device according to the present invention, the analyte flows essentially perpendicularly through a (micro)porous substrate, such as a membrane comprising immobilized capture probes on both sides of the substrate. The high specific surface areas of the porous membrane causes a large number of specifically bonded probe molecules on the detection spots, resulting in a high sensitivity for the detection of bonded molecules. In general, the porous membrane may be made from any of a variety of materials through which the test sample is capable of passing. For example, the materials used to form the porous membrane may include, but are not limited to, natural, synthetic, or naturally occurring materials that are synthetically modified. The size and shape of the porous membrane may generally vary as is readily recognized by those skilled in the art.

In order to be detected, the analyte of interest has to bind specifically to the corresponding capture probes. Capture probes may be deposited on a surface of the membrane to attract and/or capture particles. As used herein, the step of capturing an analyte to a capture probe will refer to any method which allows capturing, in particular immobilized capturing, or adhering the particles to the capture probe, e.g., to the surface of the membrane. The capture probes generally comprise receptor molecules which are capable of binding with biomolecular species of the particle or analyte to be detected, such as nucleic acids, proteins, cells or drugs present within the sample. For example, for detecting whether an antigen is present in the sample, antibody binding may be used. For detecting the presence of DNA, protein interactions may be applied. For capturing proteins, small molecules, cells, WBC, RBC or bacteria capture molecules, such as antibodies, may be deposited on the surface of the membrane.

After capturing the different analytes or particles, various detection methods can be used to sense whether the specific binding with the capture probe has occurred. Commonly, an optical detection method is used that is based on, for example, fluorescence, refractive index changes or spectral changes. Also, changes in localized magnetic or dielectric behavior are being used. In a particular example of an optical capture probe, a membrane is provided with an array of capture targets of different receptor molecules that are, for example, applied via inkjet printing and/or contact printing. For instance, for determining whether the specific capturing took place, fluorescent labeled ssDNA fragments or antibodies may be used which will bind to the capture probe-ligand complex. The presence of the specific biomolecules can then be detected by means of an optical detection device, such as a CCD camera if the fluorescent probes are excited to emit light.

The present invention provides a device for capturing first and second analytes in a test sample, wherein the (physical) size of the first analyte is larger than the (physical) size of the second analyte. The device comprises (a) a chamber; (b) a substrate or porous membrane with a first surface and a second surface, said substrate or porous membrane separates the chamber into a first and a second sub-chamber; and (c) means for supplying the test sample into the first sub-chamber. The substrate or porous membrane (1) is adapted to pass the second analyte but not to pass the first analyte and to capture the first analyte on the first surface and the second analyte on the second surface.

The first surface comprises preferably a first capture probe for immobilized capturing of the first analyte. The second surface comprises preferably second capture probe for immobilized capturing of the second analyte.

The first analyte are preferably cells, in particular WBC, RBC or bacteria and the second analyte are preferably proteins or small molecules. The first capture probe on the first surface of the substrate or membrane may be accordingly adapted to attract and/or capture cells, in particular WBC, RBC or bacteria. The second capture probe on the second surface of the substrate or membrane is preferably adapted to attract and/or capture proteins or small molecules.

The second capture probe may be selected from a group comprising nucleic acids, a protein, an antibody, a tissue, a cell, and a drug.

The device according to the present invention may further comprise means for optically visualizing the antibody-antigen interaction.

The device according to the present invention may further comprising a passage in fluid communication with the second sub-chamber.

The present invention also provides a method for capturing first and second analytes in a test sample, wherein the first analyte is larger than the second analyte. The method comprises the following steps:
- providing a substrate or a porous substrate/membrane which allows the passage of the second analyte and captures the first analyte at a first surface and the second analyte at a second surface of the substrate/membrane;
- providing the test sample at the first surface of the substrate or porous membrane and
- passing the test sample from a side of the first surface through the substrate/membrane to the side of the second surface.

The method may further comprise the step of passing back the test sample from the second surface side through the substrate/membrane to the first surface side.

The method according to the present invention may also comprise the steps of alternately pressurizing the test sample on the first surface side and on the second surface side for alternately pumping the test sample through the substrate/membrane from the first surface to the second surface and vice versa.

These and other aspects of the present invention will be apparent from the elucidated with reference to the embodiments described hereinafter.

### BRIEF DESCRIPTION OF THE DRAWINGS

Fig. 1 schematically shows a cross-sectional of a substrate according to the present invention.
Figs. 2a and 2b schematically show different types of assay modules that may be employed according to the present invention.
Fig. 3 schematically shows an embodiment of a device according to the present invention in four different stages during a pump cycle.

### DETAILED DESCRIPTION OF EMBODIMENTS

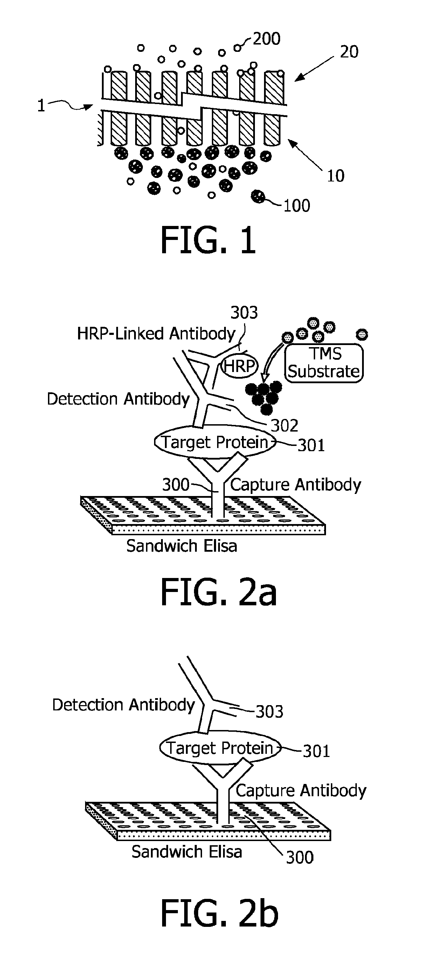

Fig. 1 schematically shows an embodiment of a substrate or membrane 1 according to the present invention. The membrane 1 comprises a first surface 10 (lower surface) and a second surface 20 (upper surface). Fig. 1 illustrates the case in which a test sample is provided under pressure as a fluid with two different analytes or particles 100 and 200. The size of the first analyte 100 is larger than the size of the second analyte 200. Moreover, the size of the first analyte is larger than the pore size of the porous membrane such that the liquid with the second analyte 200 passes through the porous membrane 1 but the first analyte is not able to pass through the membrane and will remain on the lower side of the membrane.

For instance, the first analyte consists of larger cells 100 and the second analyte consists of smaller proteins 200, both provided in the test sample of whole blood. The lower or first side 10 of the membrane comprises immobilized first capture probes or targets which are adapted to attract and/or capture the first cells 100 on the membrane. Thus, the cells are retained on the lower side of the membrane while the serum/plasma containing the blood with the proteins 200 passes through the membrane 1. Antibodies that are specific to the cells surface receptors can be immobilized on the lower surface to capture the cells of interest. Further, antibodies are provided on the upper surface 20 of the membrane 1 to immobilize the proteins 200 from the test sample and to bind the proteins to the upper surface of the membrane.

The presence or quantity of antibody-antigen interaction will be visualized, e.g., via optical techniques, as mentioned above. Thus, after capturing standard (immuno)assay can be performed.

Fig 2a schematically a secondary sandwich assay module that may be employed by the present invention. Reference sign 301 refers to a human leukocyte, 302 to a monoclonal, 301 to a biotynylated monoclonal and 300 refers to a polyclonal Ab (antibody). Fig 2b is similar to Fig. 2a but schematically shows a primary sandwich assay module that may be employed according to the present invention. In contrast to Fig. 2a, there is no monoclonal Ab 302 in Fig. 2b. Reference sign 300 refers to a polyclonal Ab, 301 to a human leukocyte and 301 to a biotynylated monoclonal.

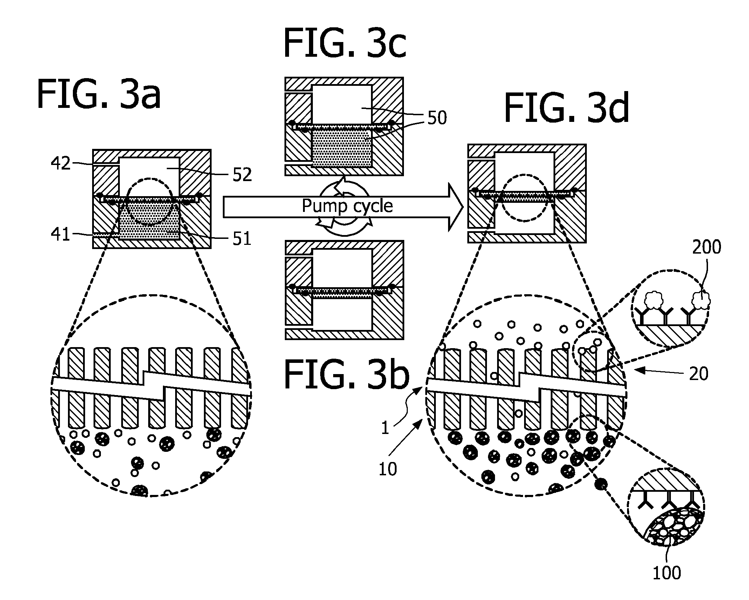

Figs. 3 a) to d) schematically show an embodiment of a flow-through assay device according to the present invention at four different stages of a pump cycle. The device comprises a porous membrane 1 which divides an analysis chamber 50 into two sub-chambers 51 and 52. In this particular embodiment, the first sub-chamber 51 is located below the porous membrane 1 and the second sub-chamber 52 is located above the porous membrane 1. The test sample to be analyzed is whole blood which comprises two different analytes, large cells and smaller proteins. The fluid is supplied to the first sub-chamber 51 through the first inlet 41 which is in fluid communication with the sub-chamber 51 (see Fig. 3a). The whole blood is provided under either pressure or vacuum, e.g., by means of a pump, and pushed through the porous membrane. The larger cells 100, which are larger than the proteins 200 of the serum/plasma, cannot pass through the pores of the porous membrane 1 (see also Fig. 1) and are retained in the first sub-chamber or inlet chamber 51. However, due to the flow of the liquid, the cells will be forced to the lower surface of the membrane and captured by the capture probes which are provided on the lower surface of the membrane (see Fig. 3b).

The second type of particles 200, namely the proteins, are smaller than the cells 100 and can pass through the pores of the membrane. In other words, the serum/plasma is pushed together with the liquid through the membrane 1 into the upper or second sub-chamber 52 (see Fig. 3b).

By inverting the flow direction through the membrane 1, e.g. by sucking the fluid back through the first inlet 41, or by providing a pressure at the second inlet 42 which is in fluid communication with the second (upper) sub-chamber 52, the smaller particles, namely the proteins, are pushed from the second sub-chamber 52 toward the second surface 20 of the membrane 1. Accordingly, the capture probes provided on the upper surface 20 of the membrane capture the proteins and bind them to the membrane (see Fig. 3c)).

Subsequently, the remaining fluid is removed such that the cells are bound to the lower surface of the membrane 1 and the proteins are bound to the upper surface of the membrane. The presence or quantity of the cells and the proteins at the membrane can be visualized, e.g., via optical techniques, as mentioned above. Thus, after capturing the cells and the proteins, known standard (immuno)assay can be performed.

By printing appropriate arrays of capture probes (e.g. antibodies) on both surfaces of the membrane, the larger cells and smaller proteins can be captured on either side of the membrane. Since the cells are retained on the lower surface, the analytes in the plasma/serum can be pumped backwards and forwards through the membrane to increase reaction kinetics and decrease assay time.

With the device and the method of the present invention, it is also possible to combine pathogen detection (either measuring the DNA or the pathogen itself) with a protein assay, e.g. sepsis, coronary artery disease, and in oncology leukemia.

While the invention has been illustrated and described in detail in the drawings and foregoing description, such illustration and description are to be considered illustrative or exemplary and not restrictive; the invention is not limited to the disclosed embodiments. Variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in different mutually dependent claims does not indicate that a combination of these measures cannot be used to advantage. Any reference signs in the claims should not be construed as limiting the scope.

## Claims

1. A device for capturing first (100) and second (200) analytes in a test sample, wherein the size of the first analyte (100) is larger than the size of the second analyte (200), said device comprising:
(a) a chamber (50);
(b) a porous substrate (1) with a first surface (10) and a second surface (20), said porous substrate (1) separating the chamber (50) into a first and a second sub-chamber (51 and 52, respectively); and
(c) means (41) for supplying the test sample into the first sub-chamber (51); wherein the porous substrate (1) is adapted to pass the second analyte but not to pass the first analyte and to capture the first analyte (100) on the first surface (10) and the second analyte (200) on the second surface (20).

2. The device according to claim 1, wherein the first surface (10) comprises a first capture probe for immobilized capturing of the first analyte (100).

3. The device according to claim 1, wherein the second surface (20) comprises second capture probe for immobilized capturing of the second analyte (200).

4. The device according to claim 1, wherein the first analyte are cells, in particular WBC, RBC or bacteria and the second analyte are proteins or small molecules.

5. The device according to claim 2, wherein the first capture probe on the first surface (10) of the substrate (1) is adapted to attract and/or capture cells, in particular WBC, RBC or bacteria.

6. The device according to claim 3, wherein the second capture probe on the second surface (20) of the substrate (1) is adapted to attract and/or capture proteins or small molecules.

7. The device according to claim 6, wherein the second capture probe is selected from a group comprising nucleic acids, a protein, an antibody, a tissue, a cell, and a drug.

8. The device according to claim 7, comprising means for optically visualizing the antibody-antigen interaction.

9. The device according to claim 1, further comprising a passage (42) in fluid communication with the second sub-chamber (52).

10. A method for capturing first (100) and second (200) analytes in a test sample, wherein the first analyte (100) is larger than the second analyte (200), comprising the following steps:
a) providing a porous substrate (1) which allows the passage of the second analyte (100) and captures the first analyte at a first surface (10) and the second analyte at a second surface (20) of the substrate (1);
b) providing the test sample at the first surface (10) of the porous substrate (1) and
c) passing the test sample from a side (51) of the first surface (10) through the substrate (1) to the side (52) of the second surface (20).

11. The method according to claim 10 further comprising the step of passing back the test sample from the second surface side (52) through the substrate (1) to the first surface side (51).

12. The method according to claim 11 comprising the steps of alternately pressurizing the test sample on the first surface side (51) and on the second surface side (52) for alternately pumping the test sample through the substrate (1) from the first surface (10) to the second surface (20) and vice versa.
